(19) Europäisches Patentamt
European Patent Office
Office européen des brevets

(11) Veröffentlichungsnummer: **0 026 867**
**A1**

(12) # EUROPÄISCHE PATENTANMELDUNG

(21) Anmeldenummer: **80105637.5**

(22) Anmeldetag: **19.09.80**

(51) Int. Cl.³: **A 61 K 31/66**, C 07 F 9/38, C 07 C 103/52, A 61 K 37/02

(30) Priorität: **28.09.79 GB 7933700**
**07.08.80 GB 8025806**

(43) Veröffentlichungstag der Anmeldung: **15.04.81**
**Patentblatt 81/15**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Atherton, Frank Ratcliffe, 148 Park Way, Welwyn Garden City, Herts. (GB)**
Erfinder: **Hall, Michael John, 15 Cannonsfield Road, Welwyn, Herts. (GB)**
Erfinder: **Hassall, Cedric Herbert, 23 The Ryde, Hatfield, Herts. (GB)**
Erfinder: **Lambert, Robert Wilson, 6 Grange Hill, Welwyn, Herts. (GB)**
Erfinder: **Ringrose, Peter Stuart, 10 Geneegasse, A-1130 Wien (AT)**

(74) Vertreter: **Lederer, Franz, Dr. et al, Patentanwälte Dr. Franz Lederer Reiner F. Meyer Lucile-Grahn-Strasse 22, D-8000 München 80 (DE)**

(54) **Peptidylphosphonsäuren, deren Herstellung und Verwendung und sie enthaltende pharmazeutische Präparate.**

(57) Peptidylphosphonsäuren der allgemeinen Formel

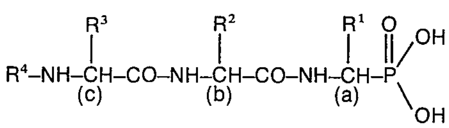

I

worin $R^1$ Wasserstoff oder Methyl ist;

$R^2$ und $R^3$ jeweils einen Niederalkylrest, der charakteristisch ist für eine in Proteinen normalerweise nicht vorkommende α-Aminosäure oder einer der beiden Substituenten einen solchen Niederalkylrest und der andere einen für eine normalerweise in Proteinen vorkommende α-Aminosäure charakteristischen Niederalkylrest darstellen;

$R^4$ der L-Pyroglutamylrest ist; die Konfiguration am C-Atom (a) R ist (sofern $R^1 \neq H$), während die Konfigurationen an den C-Atomen (b) und (c) L sind (sofern $R^2$ bzw. $R^3 \neq H$),

und deren physiologisch verträgliche Salze, deren Herstellung und Verwendung als antibakterielle Mittel sowie diese enthaltende pharmazeutische Präparate.

EP 0 026 867 A1

0026867

F.Hoffmann-La Roche & Co. Aktiengesellschaft,Basel,Schweiz

19. Sep. 1980

RAN 4105/57

# Peptidylphosphonsäuren, deren Herstellung und Verwendung und sie enthaltende pharmazeutische Präparate.

Die vorliegende Erfindung betrifft neue Phosphonsäuren bzw. Peptidderivate, ein Verfahren zu deren Herstellung sowie pharmazeutische Präparate auf der Basis dieser neuen Verbindung und deren Herstellung. Schliesslich betrifft die vorliegende Erfindung die Verwendung der neuen Verbindungen.

Bei den erfindungsgemässen Verbindungen handelt es sich um Peptidderivate der allgemeinen Formel

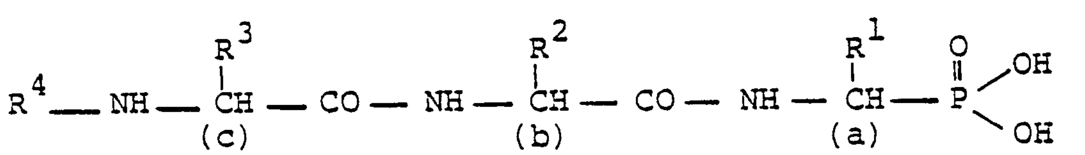

I

worin $R^1$ Wasserstoff oder Methyl ist;

$R^2$ und $R^3$ jeweils einen Niederalkylrest, der charakteristisch ist für eine in Proteinen normalerweise nicht vorkommende α-Aminosäure oder einer der beiden Substituenten einen solchen Niederalkylrest und der andere einen für eine normalerweise

in Proteinen vorkommende α-Aminosäure charakteristischen Niederalkylrest darstellen;

$R^4$ der L-Pyroglutamylrest ist;

die Konfiguration am C-Atom (a) R ist (sofern $R^1 \neq H$), während die Konfigurationen an den C-Atomen (b) und (c) L sind (sofern $R^2$ bzw. $R^3 \neq H$),

und um deren physiologisch verträgliche Salze.

In der vorliegenden Anmeldung und den dazugehörigen Ansprüchen bedeutet der Ausdruck "Niederalkyl" gerad- oder verzweigtkettige Alkylreste mit vorzugsweise bis zu 8 Kohlenstoffatomen, wie beispielsweise Ethyl, Propyl, Butyl, tert.Butyl, Pentyl und Hexyl. Bei dem charakteristischen Rest einer normalerweise in Proteinen vorkommenden bzw. nicht vorkommenden Aminosäure handelt es sich um den Substituenten R einer natürlichen α-Aminosäure der allgemeinen Formel

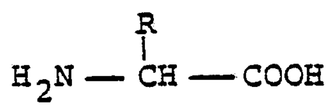

Handelt es sich bei der Aminosäure um Alanin, so ist R Methyl, handelt es sich um Methionin, so ist R 2-Methylthioethyl, beim Serin ist R Hydroxymethyl und im Tyrosin ist R p-Hydroxybenzyl, usw. R kann auch zusammen mit dem Stickstoffatom der Aminogruppe zum Ring geschlossen sein, wie im Prolin.

Wenn $R^1$ in Verbindungen der Formel I Methyl darstellt, dann soll die Konfiguration am mit (a) bezeichneten C-Atom R sein, d.h. die Konfiguration soll so sein, wie sie beim Ersatz der Carboxylgruppe einer natürlichen α-Aminosäure durch eine $PO_3H_2$-Gruppe erhalten wird.

Verbindungen der Formel I, in denen $R^1$ Methyl darstellt, sind bevorzugt. Ferner sind Verbindungen bevorzugt, in denen $R^2$ und $R^3$ Niederalkylreste darstellen, die

charakteristisch sind für normalerweise in Proteinen nicht vorkommende α-Aminosäuren.

Beispiele von erfindungsgemässen Verbindungen der Formel I sind:

(1R)-1-(L-Pyroglutamyl-L-norvalyl-norvalylamino)-ethylphosphonsäure,
(L-Pyroglutamyl-L-norvalyl-L-norvalylamino)-methylphosphonsäure,
(1R)-1-[L-Pyroglutamyl-L-(2-aminoheptanoyl)-L-norvalylamino)-ethylphosphonsäure und
(1R)-1-(L-Pyroglutamyl-L-alanyl-L-norvalylamino)-ethylphosphonsäure.

Die Verbindungen der Formel I und ihre physiologisch verträglichen Salze können erfindungsgemäss dadurch hergestellt werden dass man eine Verbindung der allgemeinen Formel

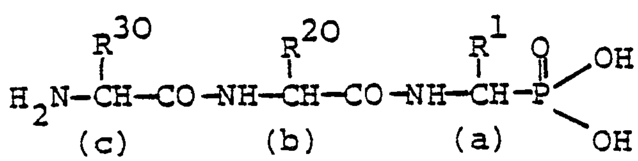

II

worin $R^1$ wie in Anspruch 1 definiert ist;
$R^{20}$ und $R^{30}$ die in Anspruch 1 für $R^2$ und $R^3$ angegebenen Bedeutungen haben, wobei Aminogruppen jedoch durch eine hydrogenolytisch abspaltbare Schutzgruppe geschützt sind
und die Konfigurationen an den C-Atomen (a), (b) und (c) wie in den Verbindungen der Formel I sind,
mit einer Verbindung der Formel

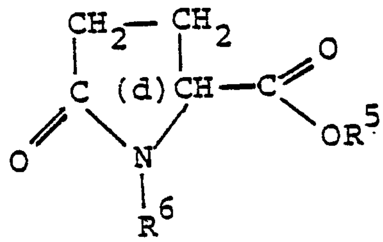

III

worin $R^5$ 2,4,5-Trichlorphenyl, Pentachlorphenyl oder Succinimidyl ist;
$R^6$ Wasserstoff oder eine Aralkoxycarbonylgruppe darstellt und die Konfiguration am C-Atom (d) L ist,
umsetzt, ein Reaktionsprodukt, das eine geschützte Aminogruppe und/oder eine Aralkoxycarbonylgruppe enthält, der Hydrogenolyse unterwirft und eine so erhaltene Verbindung gewünschtenfalls in ein physiologisch verträgliches Salz überführt.

Beispiele hydrogenolytisch abspaltbarer Aminoschutzgruppen, die in den Substituenten $R^{20}$ und $R^{30}$ einer Verbindung II vorkommen können, sind Aralkoxycarbonylgruppen, wie z.B. Benzyloxycarbonyl. Eine Aminogruppe kann auch durch eine Nitrogruppe geschützt werden, wie im Falle der Nitroarginylgruppe.

Die Umsetzung einer Verbindung II mit einer Verbindung III kann in an sich bekannter Weise durchgeführt werden, beispielsweise nach der Methode der aktivierten Ester. Die Umsetzung kann beispielsweise in einem organischen Lösungsmittel, wie wässrigem Dimethylformamid, bei etwa 0°C durchgeführt werden. In einer bevorzugten Ausführungsform wird eine Verbindung der Formel II mit einer Verbindung der Formel III, in der $R^5$ die 2,4,5-Trichlorphenylgruppe darstellt, kondensiert.

Ein erhaltenes Kondensationsprodukt, das eine geschützte Aminogruppe und/oder eine Aralkoxycarbonylgruppe $R^6$ enthält, wird der Hydrogenolyse unterworfen, um die geschützte Aminogruppe in eine Aminogruppe zu überführen und/ oder die Aralkoxycarbonylgruppe abzuspalten. Diese Hydrogenolyse kann in an sich bekannter Weise durchgeführt werden, beispielsweise in Gegenwart eines Edelmetall-Katalysators wie Palladium/C oder Platinoxid.

Physiologisch verträgliche Salze bilden die Verbindungen der Formel I mit physiologisch verträglichen, starken organischen und anorganischen Säuren (z.B. HCl, HBr, $H_2SO_4$, Methansulfonsäure, p-Toluolsulfonsäure) und Basen (z.B. NaOH, KOH).

Die Ausgangsverbindungen der Formel II sind bekannte Verbindungen.

Die Ausgangsverbindungen der Formel III können beispielsweise durch Umsetzung von L-Pyroglutaminsäure oder einer N-Aralkoxycarbonyl-L-pyroglutaminsäure mit 2,4,5-Trichlorphenol, Pentachlorphenol oder N-Hydroxysuccinimid in Gegenwart von N,N'-Dicyclohexylcarbodiimid in einem inerten organischen Lösungsmittel, wie Dimethylformamid, hergestellt werden. Wird von einer N-Aralkoxycarbonyl-L-pyroglutaminsäure ausgegangen, so wird die Verbindung der Formel III, in der $R^6$ Aralkoxycarbonyl darstellt, in an sich bekannter Weise, wie oben bereits ausgeführt, der Hydrogenolyse unterworfen. Wird eine Verbindung der Formel III verwendet, in der $R^5$ eine Succinimidylgruppe darstellt und $R^6$ Wasserstoff ist, so wird diese vorzugsweise im Reaktionsmedium in situ gebildet.

Die erfindungsgemässen Peptidderivate besitzen antibakterielle Aktivität gegen eine Vielzahl Gram-positiver und Gram-negativer Bakterien, wie z.B. Escherichia coli, Klebsiella spp., Serratia marcescens, Staphylococcus albus, Salmonella spp., Shigella spp., Streptococcus faecalis, Streptococcus pyogenes, Streptococcus pneumoniae und Haemophilus influenzae. Die Verbindungen können demgemäss zur Behandlung und Prophylaxe bakterieller Infektionen verwendet und parenteral verabreicht werden.

Die folgende Tabelle gibt die Resultate von in vitro Versuchen unter Verwendung von (1R)-1-(L-Pyroglutamyl-L-norvalyl-L-norvalylamino)-ethylphosphonsäure gegen einige repräsentative pathogene Mikroorganismen wieder.

Tabelle

| Mikroorganismus | M.I.C. (µg/ml) |
|---|---|
| E. coli | 0.25 |
| Klebsiella spp. | 16 |
| S. marcescens | 30 |
| S. albus | 16 |
| Salmonella spp. | 20 |
| Shigella spp. | 1.1 |
| S. faecalis | 0.05 |
| S. pyogenes | 16 |
| S. pneumoniae | 2.4 |
| H. influenzae | 0.2 |

Darüberhinaus potenzieren die erfindungsgemässen Peptidderivate die Aktivität von Antibiotika, vorzugsweise von β-Lactam-Antibiotika wie Penicillinen, Cephalosporinen oder monocyclischen β-Lactam-Antibiotika.

Beispiele geeigneter Penicilline sind Ampicillin, Carbenicillin, Penicillin G, Sulbenicillin, Mecillinam, Pivmecillinam, Phenethicillin, Methicillin, Propicillin, Ticarcillin, Amoxycillin und Piperacillin.

Geeignete Cephalosporine sind Cephalexin, Cephazolin, Cefoxitin, Cephradin, Cefsulodin, Cephamandol, Cephaloridin, Cephaloglycin, Cefatrizin, Cefacetril, Cefuroxim, Cefaclor, Cefotaxim, Cefazedon, wobei Cephalexin bevorzugt ist.

Beispiele anderer Antibiotika sind D-Cycloserin, Rifampicin, Phosphonomycin, Gentamycin, Vancomycin und Kanamycin.

Bei den vorstehend aufgezählten Antibiotika handelt es sich um bekannte Verbindungen, die nach bekannten Methoden hergestellt werden können.

Die vorliegende Erfindung betrifft daher auch pharmazeutische Präparate, die neben einem Peptid der Formel I ein Antibiotikum enthalten.

Die Herstellung der pharmazeutischen Präparate kann in an sich bekannter Weise geschehen, dadurch z.B., dass man die einzelnen Komponenten mit den geeigneten Trägermaterialien vermischt und in eine geeignete galenische Form bringt. Als Trägermaterialien kommen feste oder flüssige Substanzen in Frage, die mit den Peptidderivaten und den Antibiotika verträglich sind. Es kann sich um organische oder anorganische Substanzen handeln, die sich für eine enterale Verabreichung eignen. Beispiele solcher Trägermaterialien sind Wasser, Gelatine, Mannit, mineralische und vegetabile Oele, Gummi arabicum, Propylenglycol und Polyalkylenglycole. Die pharmazeutischen Präparate können in fester Form (z.B. als Lyophilisate) oder in flüssiger Form (z.B. als Lösungen, Suspensionen oder Emulsionen) vorliegen. Bei der Herstellung können sie den üblichen pharmazeutischen Operationen, wie Sterilisation, unterworfen werden. Schliesslich können sie Zusatzstoffe, wie Konservierungs-, Stabilisierungs-, Netz- oder Emulgiermittel, Salze zur Veränderung des osmotischen Druckes oder Puffer enthalten. Bei Verwendung eines Puffers kann das pH des pharmazeutischen Präparates natürlich innerhalb des aus der pharmazeutischen Praxis wohlbekannten Bereiches variieren.

Das Gewichtsverhältnis von Peptidderivat der Formel I zu Antibiotikum kann innerhalb weiter Grenzen variieren. Es liegt zweckmässigerweise im Bereich von 1:100 bis 100:1, vorzugsweise im Bereich von 1:64 bis 64:1. Besonders bevorzugt ist ein Gewichtsverhältnis von 1:16 bis 16:1.

Die tägliche Dosierung der Peptidderivate, sei es alleine oder in Kombination mit einem Antibiotikum, kann innerhalb weiter Grenzen variieren, und zwar in Abhängigkeit von Faktoren, wie dem speziell gewählten Peptidderivat bzw. Antibiotikum, der zu behandelnden Infektion, usw. So kann z.B. die tägliche Dosis für ein Peptidderivat allein bei parenteraler Applikation 800-200 mg betragen. Wird das Peptidderivat in Kombination mit einem Antibiotikum verabreicht, so kann die tägliche Dosis bei parenteraler Applikation etwa 200-2000 mg betragen. Es versteht sich, dass die tägliche Dosis als Einzeldosis oder in mehreren Teilmengen verabreicht werden kann und dass die erwähnten Dosierungen sowohl nach unten wie nach oben variiert werden können, je nach individuellen Erfordernissen und in Anpassung an die besonderen Umstände, wie sie vom behandelnden Arzt angetroffen werden.

Die folgenden Beispiele erläutern die Erfindung.

## Beispiel 1

8,0 g (25 mMol) (1R)-1-(L-Norvalyl-L-norvalylamino)-ethylphosphonsäure wurden unter Rühren in einem Gemisch aus 125 ml Wasser und 5,1 g (50 mMol) Triethylamin gelöst. Es wurden 125 ml Dimethylformamid zugesetzt und die Lösung auf 0°C abgekühlt. Nach Zusatz von 20,3 g (66 mMol) L-Pyroglutaminsäure-2,4,5-trichlorphenylester und 125 ml Dimethylformamid bei 0°C wurde unter langsamem Erwärmen des Kühlbades auf Zimmertemperatur gerührt. Die erhaltene Suspension wurde filtriert und der gelatinöse Niederschlag mit wässrigem Dimethylformamid gewaschen. Die vereinigten Filtrate wurden unter vermindertem Druck zur Trockene eingeengt und der Rückstand mit 200 ml Diethylether behandelt. Der Diethylether wurde entfernt und der Rückstand in einem Gemisch aus 500 ml Methanol und 100 ml Wasser gelöst. Nach Zusatz von 200 g Zerolit 225, SRC 13, $RSO_3H$, und 5-minütigem Rühren wurde abfiltriert. Das Ionenaustauscherharz wurde mit Wasser und Methanol gewaschen. Das mit dem Filtrat vereinigte Waschwasser wurde zur Trockene eingeengt, der Rückstand mit 250 ml Aceton behandelt und bei Raumtemperatur einige Stunden gerührt. Der Niederschlag wurde abfiltriert und lieferte nach Waschen mit Aceton und Diethylether 4,5 g (1R)-1-(L-Pyroglutamyl-L-norvalyl-L-norvalylamino)-ethylphosphonsäure, F. 260°C (Zers.); $[\alpha]_D^{20}$ = -84,5° (c = 0,55% in Trifluoressigsäure).

## Beispiel 2

In zu Beispiel 1 analoger Weise wurden aus 9,27 g (30 mMol) (L-Norvalyl-L-norvalylamino)-methylphosphonsäure und 18,5 g (60 mMol) L-Pyroglutaminsäure-2,4,5-trichlorphenylester nach Behandlung mit Aceton 9,1 g rohe L-Pyroglutamyl-L-norvalyl-L-norvalylamino)-methylphosphonsäure erhalten. Umkristallisation aus Wasser/Ethanol (1:4, v/v) lieferte einen Schmelzpunkt von 230-232°C (Zers.); $[\alpha]_D^{20}$ = -69,6°; $[\alpha]_{365}^{20}$ = -222° (c = 0,5%

in Wasser).

## Beispiel 3

In zu Beispiel 1 analoger Weise, aber unter Verwendung von Methanol/Wasser (2:1, v/v) in der Ionenaustauscher-Stufe, wurden aus 4,2 g (12 Mmol) (1R)-1-[(L-2-Amino-heptanoyl)-L-norvalylamino]-ethylphosphonsäure und 9,3 g (30 mMol) L-Pyroglutaminsäure-2,4,5-trichlorphenylester 1,64 g rohe (1R)-1-[L-Pyroglutamyl-L-(2-aminoheptanoyl)-L-norvalylamino]-ethylphosphonsäure mit einem Schmelzpunkt von 225-230°C (Zers.), $[\alpha]_D^{20}$ = -63,9°C, $[\alpha]_{365}^{20}$ = 208° (c = 0,48% in Trifluoressigsäure), erhalten. Die Reinigung durch Rühren mit Methanol bei Raumtemperatur, Filtration und Waschen mit Diethylether lieferte reines Produkt; F. 235-237°C (Zers.); $[\alpha]_D^{20}$ = -70,6°; $[\alpha]_{365}^{20}$ = -232° (c = 0,5% in Trifluoressigsäure).

Die als Ausgangsmaterial verwendete (1R)-1-[(L-2-Aminoheptanoyl)-L-norvalylamino]-ethylphosphonsäure wurde folgendermassen hergestellt:

a) 3,6 g (25 mMol) L-2-Aminoheptansäure (J. Biol. Chem. 203, 333 (1953)] wurden unter Rühren in 25 ml (25 mMol) 1N Natriumhydroxid gelöst. Die erhaltene Lösung wurde auf 0°C abgekühlt und abwechselnd in je 5 Portionen mit insgesamt 10 ml (40 mMol) 4N Nariumhydroxid und 6,5 g (38 mMol) Benzylchlorformiat innerhalb von 30 Minuten versetzt, wobei die Temperatur auf 0-10°C gehalten wurde. Das Gemisch wurde weitere 4 Stunden gerührt unter langsamer Erwärmung auf Zimmertemperatur. Dann wurde mit Diethylether extrahiert, die Etherextrakte wurden mit Wasser gewaschen, die vereinigten wässrigen Extrakte durch Zusatz von 6N HCl auf pH 2,0 angesäuert und dreimal mit Diethylether extrahiert. Die Extrakte wurden über Natriumsulfat getrocknet, filtriert und zu 7,8 g N-Benzyloxycarbonyl-L-2-aminoheptansäure in Form eines Oeles eingeengt.

 

b) Das erhaltene Oel wurde unter Rühren in 75 ml Dimethoxyethan aufgenommen, mit 2,9 g (25 mMol) N-Hydroxysuccinimid und nach Abkühlung auf 0°C mit 5,6 g (27,5 mMol) Dicyclohexylcarbodiimid versetzt. Das Gemisch wurde bei 0°C gerührt und bei dieser Temperatur über Nacht stehengelassen. Das feste Nebenprodukt (5,9 g Dicyclohexylharnstoff) wurde abfiltriert, das Filtrat zu einem Oel eingeengt und mit Petrolether behandelt. Der erhaltene weisse Feststoff wurde abfiltriert und lieferte 9,2 g N-Benzyloxycarbonyl-L-2-aminoheptansäure-N-hydroxysuccinimid, F. 65-67°C; $[\alpha]_D^{20}$ = -23,,6°; $[\alpha]_{365}^{20}$ = -80,9° (c = 1% in Ethanol).

c) 4,48 g (20 mMol) (1R)-1-(L-Norvalylamino)-ethylphosphonsäure wurden unter Rühren in einem Gemisch aus 50 ml Wasser und 4,04 g (40 mMol) Triethylamin gelöst. Nach Zusatz von 50 ml Dimethylformamid wurde die Lösung auf 0°C abgekühlt, tropfenweise mit 9,2 g (24 mMol) N-Benzyloxycarbonyl-L-2-aminoheptansäure-N-hydroxysuccinimid in 50 ml Dimethylformamid versetzt, wobei das Reaktionsgemisch auf 0-5°C gehalten wurde, und allmählich auf Raumtemperatur erwärmt. Nach Filtration wurde das Filtrat eingeengt, der Rückstand mit 200 ml Wasser versetzt und die erhaltene Suspension mit ca. 20 ml 2N HCl behandelt. Der erhaltene Niederschlag wurde mit Wasser gewaschen, filtriert und mehrere Stunden mit Aceton behandelt. Ausbeute: 8,7 g (90%) (1R)-1-[[N-Benzyloxycarbonyl-L-(2 aminoheptanoyl)-L-norvalyl]-amino]-ethylphosphonsäure, F. 180-190°C (Zers.)

d) 8,7 g (18 mMol) (1R)-1-[[N-Benzyloxycarbonyl-L-(2-aminoheptanoyl)-L-norvalyl]-amino]-ethylphosphonsäure wurden mit einem Gemisch von 20 ml 45%-iger Bromwasserstoffsäure in Eisessig 4 Stunden bei Raumtemperatur gerührt. Nach Zusatz von 150 ml Diethylether wurde ein gummiartiger Niederschlag erhalten, der mit weiteren 150 ml Diethylether gewaschen und in 100 ml Methanol unter Rühren aufgenommen wurde. Nach Zusatz von 10 ml Propylenoxid

wurde ein gelatinöser Niederschlag erhalten. Es wurden weitere 100 ml Methanol zugesetzt und das Gemisch über Nacht bei Zimmertemperatur stehengelassen. Der Niederschlag wurde abfiltriert, zunächst mit Methanol, dann mit Diethylether gewaschen und lieferte 6,3 g rohe (1R)-1-[L-(2-Aminoheptanoyl)-L-norvalylamino]-ethylphosphonsäure, F. 250-255°C (Zers.). Nach Rühren mit 250 ml heissem Wasser, Abkühlen, Filtration und Waschen mit Ethanol sowie Diethylether wurden schliesslich 5,36 g Reinsubstanz erhalten, F. 268-272°C (Zers.); $[\alpha]_D^{20}$ = -25,8°; $[\alpha]_{365}^{20}$ = -89,0° (c = 0,36% in 2N HCl).

## Beispiel 4

In zu Beispiel 1 analoger Weise, aber unter Verwendung von Methanol/Wasser (1:1, v/v) in der Ionenaustausch-Stufe, wurden aus 4,65 g (15 mMol) (1R)-1-(L-Alanyl-L-norvalylamino)-ethylphosphonsäure und 11 g (35 mMol) L-Pyroglutaminsäure-2,4,5-trichlorphenylester 6,04 g eines Rohproduktes erhalten, dass nach Reinigung durch Behandlung mit Aceton 5,74 g reine (1R)-1-(L-Pyroglutamyl-L-alanyl-L-norvalylamino)-ethylphosphonsäure, F. 262-264°C (Zers.); $[\alpha]_D^{20}$= -99,6°; $[\alpha]_{365}^{20}$ = -332° (c = 0,05% in Trifluoressigsäure), lieferte.

## Beispiel 5

Injektionslösung, enthaltend:

| | | pro 1000 ml |
|---|---|---|
| Peptidderivat | | 50,0 g |
| 0,2N NaOH-Lösung q.s. | ad pH 6.5 | |
| Wasser für Injektionszwecke | ad | 1000 ml |

## Beispiel 6

Hartgelatine-Kapseln, enthaltend die folgenden Bestandteile:

| | pro Kapsel |
|---|---|
| Peptidderivat | 125,0 mg |
| Cephalexin | 125,0 mg |
| Maisstärke | 20,0 mg |
| Mikrokristalline Cellulose | 34,0 mg |
| Dioctylnatriumsulfosuccinat | 0,5 mg |
| Stearinsäure | 5,5 mg |
| | 310,0 mg |

Patentansprüche

1. Peptidylphosphonsäuren der allgemeinen Formel

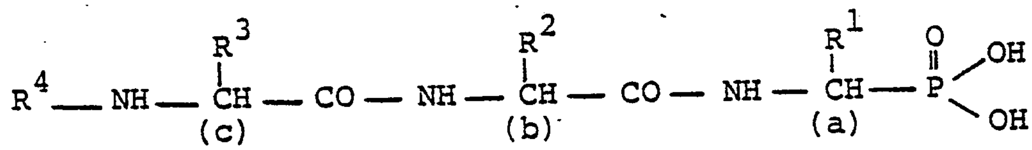 I

worin $R^1$ Wasserstoff oder Methyl ist;

$R^2$ und $R^3$ jeweils einen Niederalkylrest, der charakteristisch ist für eine in Proteinen normalerweise nicht vorkommende α-Aminosäure oder einer der beiden Substituenten einen solchen Niederalkylrest und der andere einen für eine normalerweise in Proteinen vorkommende α-Aminosäure charakteristischen Niederalkylrest darstellen;

$R^4$ der L-Pyroglutamylrest ist;

die Konfiguration am C-Atom (a) R ist (sofern $R^1 \neq H$), während die Konfigurationen an den C-Atomen (b) und (c) L sind (sofern $R^2$ bzw. $R^3 \neq H$),

und deren physiologisch verträgliche Salze.

2. Verbindungen gemäss Anspruch 1, dadurch gekennzeichnet, dass $R^1$ Methyl ist.

3. Verbindungen gemäss Anspruch 1 oder 2, dadurch gekennzeichnet, dass $R^2$ und $R^3$ jeweils einen Niederalkylrest darstellen, der charakteristisch ist für eine normalerweise in Proteinen nicht vorkommende α-Aminosäure.

4. (1R)-1-(L-Pyroglutamyl-L-norvalyl-L-norvalylamino)-ethylphosphonsäure.

5. (L-Pyroglutamyl-L-norvalyl-L-norvalylamino)-methylphosphonsäure.

6. (1R)-1-[L-Pyroglutamyl-L-(2-aminoheptanoyl)-L-norvalylamino]-ethylphosphonsäure.

7. (1R)-1-(L-Pyroglutamyl-L-alanyl-L-norvalylamino)-ethylphosphonsäure.

8. Peptidylphosphonsäuren gemäss einem der Ansprüche 1-7 als antibakterielle Mittel oder Potentiatoren von Antibiotika.

9. Verfahren zur Herstellung von Peptidylphosphonsäuren der Formel I gemäss Anspruch 1 und deren physiologisch verträglichen Salzen, dadurch gekennzeichnet, dass man eine Verbindung der allgemeinen Formel

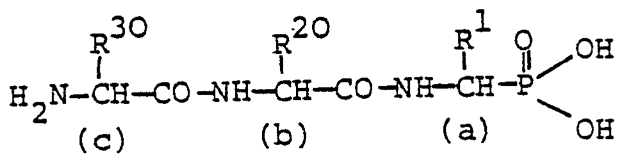

II

worin $R^1$ wie in Anspruch 1 definiert ist;

$R^{20}$ und $R^{30}$ die in Anspruch 1 für $R^2$ und $R^3$ angegebenen Bedeutungen haben, wobei Aminogruppen jedoch durch eine hydrogenolytisch abspaltbare Schutzgruppe geschützt sind

und die Konfigurationen an den C-Atomen (a), (b) und (c) wie in den Verbindungen der Formel I definiert sind, mit einer Verbindung der Formel

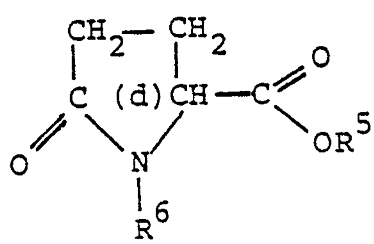

III

worin $R^5$ 2,4,5-Trichlorphenyl, Pentachlorphenyl oder Succinimidyl ist;

$R^6$ Wasserstoff oder eine Aralkoxycarbonylgruppe darstellt und die Konfiguration am C-Atom (d) L ist,

umsetzt, ein Reaktionsprodukt, das eine geschützte Aminogruppe und/oder eine Aralkoxycarbonylgruppe enthält, der Hydrogenolyse unterwirft und eine so erhaltene Verbindung gewünschtenfalls in ein physiologisch verträgliches Salz überführt.

10. Pharmazeutische Präparate auf der Basis einer Peptidylphosphonsäure gemäss einem der Ansprüche 1-7.

11. Pharmazeutische Präparate auf der Basis einer Peptidylphosphonsäure gemäss einem der Ansprüche 1-7 und einem Antibiotikum.

12. Pharmazeutische Präparate gemäss Anspruch 11, dadurch gekennzeichnet, dass das Antibiotikum ein β-Lactam-Antibiotikum ist.

13. Verwendung der Paptidylphosphonsäuren oder ihrer physiologisch verträglichen Salze gemäss einem der Ansprüche 1-7 zur Therapie und Prophylaxe bakterieller Infektionen.

***

## Patentansprüche für Oesterreich

1. Verfahren zur Herstellung von Peptidylphosphonsäuren der allgemeinen Formel

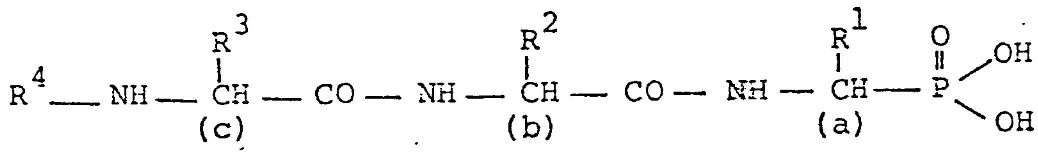

I

worin $R^1$ Wasserstoff oder Methyl ist;

$R^2$ und $R^3$ jeweils einen Niederalkylrest, der charakteristisch ist für eine in Proteinen normalerweise nicht vorkommende α-Aminosäure oder einer der beiden Substituenten einen solchen Niederalkylrest und der andere einen für eine normalerweise in Proteinen vorkommende α-Aminosäure charakteristischen Niederalkylrest darstellen;

$R^4$ der L-Pyroglutamylrest ist;

die Konfiguration am C-Atom (a) R ist (sofern $R^1 \neq H$), während die Konfigurationen an den C-Atomen (b) und (c) L sind (sofern $R^2$ bzw. $R^3 \neq H$),

und von deren physiologisch verträglichen Salzen, dadurch gekennzeichnet, dass man eine Verbindung der allgemeinen Formel

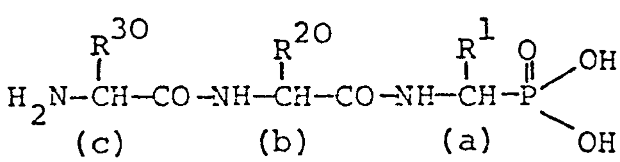

II

worin $R^1$ wie oben definiert ist;

$R^{20}$ und $R^{30}$ die oben für $R^2$ und $R^3$ angegebenen Bedeutungen haben, wobei Aminogruppen jedoch durch eine hydrogenolytisch abspaltbare Schutzgruppe geschützt sind und

die Konfigurationen an den C-Atomen (a), (b) und (c) wie in den Verbindungen der Formel I sind,

mit einer Verbindung der Formel

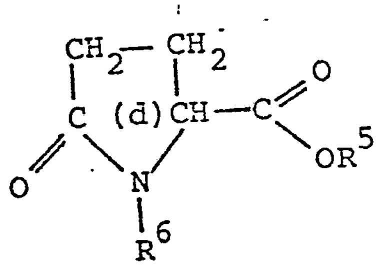

III

worin $R^5$ 2,4,5-Trichlorphenyl, Pentachlorphenyl oder Succinimidyl ist;

$R^6$ Wasserstoff oder eine Aralkoxycarbonylgruppe darstellt und die Konfiguration am C-Atom (d) L ist,

umsetzt, ein Reaktionsprodukt, das eine geschützte Aminogruppe und/oder eine Aralkoxycarbonylgruppe enthält, der Hydrogenolyse unterwirft und eine so erhaltene Verbindung gewünschtenfalls in ein physiologisch verträgliches Salz überführt.

2. Verfahren gemäss Anspruch 1, dadurch gekennzeichnet, dass in der Ausgangsverbindung der Formel II $R^1$ Methyl ist.

3. Verfahren gemäss Anspruch 1 oder 2, dadurch gekennzeichnet, dass in der Ausgangsverbindung der Formel II $R^{20}$ und $R^{30}$ jeweils einen Niederalkylrest darstellen, der für eine in Proteinen normalerweise nicht vorkommende α-Aminosäure charakteristisch ist.

4. Verfahren gemäss einem der Ansprüche 1-3, dadurch gekennzeichnet, dass die Ausgangsverbindung der Formel II

(1R)-1-(L-Norvalyl-L-norvalylamino)-ethyl-phosphonsäure ist.

5. Verfahren gemäss einem der Ansprüche 1-3, dadurch gekennzeichnet, dass die Ausgangsverbindung der Formel II (L-Norvalyl-L-norvalylamino)-methyl-phosphonsäure ist.

6. Verfahren gemäss einem der Ansprüche 1-3, dadurch gekennzeichnet, dass die Ausgangsverbindung der Formel II (1R)-1-[L-(2-Aminoheptanoyl)-L-norvalylamino]-ethylphosphonsäure ist.

7. Verfahren gemäss einem der Ansprüche 1-3, dadurch gekennzeichnet, dass die Ausgangsverbindung der Formel II (1R)-1-(L-Alanyl-L-norvalylamino)-ethylphosphonsäure ist.

0026867

Europäisches Patentamt

# EUROPÄISCHER RECHERCHENBERICHT

Nummer der Anmeldung
EP 80 10 5637

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | DE - A - 2 730 549 (HOFFMANN)<br>* Das ganze Dokument *<br>-- | 1-3, 8-13 |
| | DE - A - 2 602 193 (HOFFMANN)<br>* Das ganze Dokument *<br>-- | 1,2,9, 10 |
| | EP - A - 0 002 822 (HOFFMANN)<br>* Das ganze Dokument *<br>-- | 1,2,9, 10 |
| P | EP - A - 0 010 872 (CIBA-GEIGY)<br>* Das ganze Dokument *<br>-- | 1,2,9, 10 |
| P | EP - A - 0 010 067 (CIBA-GEIGY)<br>* Das ganze Dokument *<br>---- | 1,2,9, 10 |

KLASSIFIKATION DER ANMELDUNG (Int. Cl.³)

A 61 K 31/66
C 07 F 9/38
C 07 C 103/52
A 61 K 37/02

RECHERCHIERTE SACHGEBIETE (Int. Cl.³)

C 07 C 103/52
C 07 F 9/38

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 15-01-1981 | RAJIC |